# EUROPEAN PATENT SPECIFICATION

(11) **EP 1 838 683 B1**
(45) Date of publication and mention of the grant of the patent: **29.04.2009**
(21) Application number: 05795620.3
(22) Date of filing: 25.10.2005
(51) Int. Cl.: C07D 273/06

(54) **A PROCESS FOR THE PREPARATION [1,4,5]-OXADIAZEPINE DERIVATIVES**
VERFAHREN ZUR HERSTELLUNG VON [1,4,5]-OXADIAZEPINDERIVATEN
PROCEDE POUR LA PREPARATION DE DERIVES DE [1,4,5]-OXADIAZEPINE

(30) Priority: 27.10.2004 CH 17762004
(43) Date of publication of application: 03.10.2007
(73) Proprietor: Syngenta Participations AG, 4058 Basel (CH)
(72) Inventor: FABER, Dominik Syngenta Crop Prot. Muenchwilen AG, CH-4333 Muenchwilen (CH); JAU, Beat Syngenta Crop Protection Muenchwilen AG, CH-4333 Muenchwilen (CH)
(74) Representative: Hölscher, Ingo
(86) International application number: PCT/EP2005/011432
(87) International publication number: WO 2006/045587

(56) References cited:
- WO-A-01/17351
- WO-A-01/17973
- WO-A-03/051853
- CORRAL, C; LISSAVETZKY, J; QUINTANILLA, G: "New Method for the Synthesis of Chloro-Substituted Dibenzo[b,f][1,4,5]thiadiazepines and their 5,6-Dihydro Derivatives" JOURNAL OF ORGANIC CHEMISTRY., vol. 47, 1982, pages 2214-2215, XP002367372 AMERICAN CHEMICAL SOCIETY, WASHINGTON, DC.

## Description

The present invention relates to a novel process for the preparation of [1,4,5]-oxadiazepines.

According to WO 03/051853, [1,4,5]-oxadiazepines can be prepared by reacting various N,N'-diacylated hydrazines with, for example, 2,2'-dichlorodiethyl ether in a polar solvent to form 4,5-diacyl-[1,4,5]-oxadiazepines and then removing the two acyl groups using a hydrohalic acid.

Surprisingly, it has now been found that the preparation of [1,4,5]-oxadiazepine derivatives can be further improved by carrying out the conversion of 4,5-diacyl-[1,4,5]-oxadiazepines into the corresponding [1,4,5]-oxadiazepines using a base.

The present invention accordingly relates to a novel process for the preparation of [1,4,5]-oxadiazepine derivatives by reaction of a 4,5-diacyl-[1,4,5]-oxadiazepine with a base in a polar solvent and at elevated temperature.

Preferred 4,5-diacyl-[1,4,5]-oxadiazepines correspond to formula I 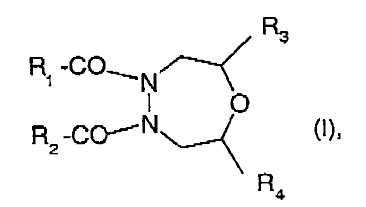 wherein R₁ and R₂ are each independently of the other hydrogen, C₁-C₅alkyl, C₁-C₅haloalkyl, C₂-C₅alkenyl, C₂-C₅alkynyl, phenyl, alkylphenyl, halophenyl, alkoxyphenyl, benzyl, alkylbenzyl, halobenzyl, alkoxybenzyl, C₁-C₅alkoxy-C₁-C₅alkyl or C₃-C₆cycloalkyl, or R₁ and R₂ together are C₁-C₄alkylene, 1,2-phenylene or 1,8-naphthylene, and R₃ and R₄ are each independently of the other hydrogen, C₁-C₅alkyl, C₁-C₅alkoxy-C₁-C₅alkyl, phenyl, alkylphenyl, halophenyl, alkoxyphenyl or benzyl.

Preferably, R₁ and R₂ are each independently of the other hydrogen or C₁-C₅alkyl, especially methyl. R₃ and R₄ are preferably hydrogen.

The 4,5-diacyl-[1,4,5]-oxadiazepines of formula I used according the invention as starting materials are known and can be prepared in a manner known *per* se, for example in the manner described in WO 03/051853. The yield of such starting materials can be improved in the case of the reaction of N,N'-diacylated hydrazines with, for example, 2,2'-dichlorodiethyl ether, by using hydroxides of alkali metals and alkaline earth metals as the base and by carrying out the reaction with the addition of a phase transfer catalyst, such as, for example, TBACI (tetrabutylammonium chloride), TBABr (tetrabutylammonium bromide), TMACI (tetramethylammonium chloride) or TMABr (tetrabutylammonium bromide) or benzyl-triethylammonium chloride, benzyl-triethylammonium bromide or Aliquat, and/or by continuously distilling off the water formed during the reaction from the reaction mixture.

An N,N'-diacylated hydrazine can be prepared by first reacting hydrazine hydrate with an acyl ester to form the monoacylated hydrazine and then, without intermediate isolation of the monoacylated hydrazine, adding an acyl anhydride to the highly concentrated aqueous-alcoholic reaction mixture. The solvents can be removed completely from the reaction mixture, for example by concentration by evaporation, and the residue can be used further without being further purified.

The alkyl radicals in the substituent definitions of the compounds of formula I contain from 1 to 5 carbon atoms and are, for example, methyl, ethyl, propyl, butyl or pentyl or branched isomers thereof. Alkoxy radicals are derived from the mentioned alkyl radicals. Alkenyl and alkynyl radicals each have from 2 to 5 carbon atoms and are, for example, ethenyl, propenyl, ethynyl and propynyl and branched isomers thereof, and also butenyl, butynyl, pentenyl, pentynyl and also branched and di-unsaturated isomers thereof. The phenyl radicals may furthermore be mono- or poly-substituted by halogen, alkyl or alkoxy, for example each of which has from 1 to 4 carbon atoms, which preferably occupy the ortho or meta position or ortho and para positions. Halogen is preferably fluorine, chlorine or bromine.

The reaction according to the invention is carried out in polar solvents, preferably in water or alcohols that preferably have a boiling point above 100°C, such as, for example, n-butanol, n-pentanol, cyclohexanol, phenol, benzyl alcohol and especially glycol, diethylene glycol, glycerol and C₁-C₄alkoxy-C₁-C₄alcohols, such as methoxyisopropanol and ethoxyethanol, and also DMSO [(CH₃)₂SO], sulfolane [(CH₂)₄SO₂], NMP [(CH₂)₃CONCH₃], DMA [CH₃CON(CH₃)₂] or DMF [HCON(CH₃)₂] or mixtures thereof, with preference being given to NMP, DMSO and, especially, water. It is also possible to use two-phase systems that contain, for example, water and an aromatic solvent, such as toluene, chlorobenzene, dichlorobenzene, xylene or anisole.

The expression "elevated temperature" preferably denotes a temperature range of from 50 to 150°C. Especially advantageously, a range of from 80 to 100°C is used.

The reaction can also be carried out under pressure, pressures of up to 10 bar preferably being used.

By adding a phase transfer catalyst, such as, for example, TBACI (tetrabutylammonium chloride), TBABr (tetrabutylammonium bromide), TMACI (tetramethylammonium chloride) or TMABr (tetrabutylammonium bromide), or benzyl-triethylammonium chloride or benzyl-triethylammonium bromide or Aliquat, the reaction can be further improved in terms of yields.

Bases suitable for the reaction according to the invention are preferably hydroxides, carbonates and alcoholates of alkali metals and alkaline earth metals, with alkali metal hydroxides being preferred. Potassium hydroxide is especially suitable. Preferably, from 1 to 2 equivalents, especially from 1 to 1.3 equivalents, of base are used per acyl group to be removed. The base can be used in solid form or can be used in solution in one of the mentioned polar solvents, for example in water in a concentration of from 10 to 70%, preferably from 40 to 65%.

The yields of isolated [1,4,5]-oxadiazepine are generally from 60 to 95%. The purity of the [1,4,5]-oxadiazepine is usually about 90%.

In the synthesis of [1,4,5]-oxadiazepine derivatives, the usual procedure is to introduce 4,5-diacyl-[1,4,5]-oxadiazepine into the polar solvent and heat the mixture. A stoichiometric amount or a suitable excess of base is then added and the reaction mixture is maintained at the selected temperature for approximately from 1 to 10 hours, preferably from 2 to 6 hours. The reaction mixture is extracted using an aromatic solvent that has poor miscibility with the reaction medium, such as chlorobenzene, at a temperature of from 20 to 100°C, preferably in the range from 60 to 80°C, thus yielding a solution comprising the [1,4,5]-oxadiazepine from which the latter can be isolated in customary manner, for example by distilling off the aromatic solvent. The extraction can be carried out batchwise or continuously.

In principle, however, it is also possible to meter in the 4,5-diacyl-[1,4,5]-oxadiazepine instead of the base, or to meter in both components, base and 4,5-diacyl-[1,4,5]-oxadiazepine.

In order to facilitate isolation of the product, a salt that is inert towards the reaction mixture and soluble therein can be added thereto. The salt used for that purpose is preferably the same salt as that obtained when the acyl group is removed, that is to say an acetate, for example potassium acetate. At a suitable salt concentration, direct separation of the [1,4,5]-oxadiazepine can in that way be achieved.

The process according to the invention can be carried out continuously or in batches (discontinuously, batchwise), with the batch procedure being preferred. The reaction times are generally from 1 to 10 hours. The batchwise reaction procedure is preferably carried out in a stirred vessel, and the continuous reaction procedure, for example, in a stirred vessel cascade.

Compared with the known removal of the acyl groups using hydrohalic acid, the process according to the invention has the following advantages:
- higher volumetric yields can be achieved since, in the case of the reaction using hydrohalic acid, a viscous crystal suspension comprising the hydrohalide of the [1,4,5]-oxadiazepine in question is formed which, at a certain concentration and above, seriously impairs the stirrability of the reaction mass
- by metering in the base and/or the 4,5-diacetyl-[1,4,5]-oxadiazepine, the reaction can be controlled in a simple manner
- the addition of the readily soluble salts enables extensive extraction of the [1,4,5]-oxadiazepine to be carried out
- reliability of the process is improved, because the thermal stability of the [1,4,5]-oxadiazepine derivatives is far better than that of the corresponding hydrohalides
- the isolation of [1,4,5]-oxadiazepines by extraction is considerably simpler than the isolation of the corresponding hydrohalides
- the cycle time is appreciably shorter

The [1,4,5]-oxadiazepine derivatives prepared according to the invention are used especially as intermediates in the preparation of herbicides of the tetrahydropyrazolodione type, which are described, for example, in WO 99/47525.

The following Examples further illustrate the invention.

### Example 1: Preparation of [1,4,5]-oxadiazepine

96.6 g of 4,5-diacetyl-[1,4,5]-oxadiazepine (content 96.5%) are introduced at from 75 to 80°C into a solution of 67.2 g of water and 100 g of potassium acetate. Then, at the same temperature, 134.4 g of aqueous 50% potassium hydroxide solution are added dropwise in the course of 30 minutes. The reaction mixture is then maintained at from 90 to 100°C for 4 hours. After cooling to from 50 to 75°C, extraction is carried out with chlorobenzene (1 x 200 g, 2 x 100 g). The combined chlorobenzene extracts contain 33.4 g of [1,4,5]-oxadiazepine, which corresponds to a yield of 65%.

### Example 2: Preparation of [1,4,5]-oxadiazepine

96.6 g of 4,5-diacetyl-[1,4,5]-oxadiazepine (content 96.5%) are introduced in the course of 15 minutes, at from 80 to 85°C, into a solution of 10.8 g of water, 100 g of potassium acetate and 123.2 g of aqueous 50% potassium hydroxide solution. The reaction mixture is then maintained at from 90 to 100°C for 4 hours. After cooling to from 50 to 75°C, extraction is carried out with chlorobenzene (1 x 200 g, 2 x 100 g). The combined chlorobenzene extracts contain 41.3 g of [1,4,5]-oxadiazepine, which corresponds to a yield of 80.9%.

### Example 3: Preparation of [1,4,5]-oxadiazepine

Batch 1: A mixture consisting of 47.2 g of water, 110 g of 98% potassium acetate and 111.0 g of 4,5-diacetyl-[1,4,5]-oxadiazepine (content 92.1 %) is prepared at from 90 to 95°C and, in the course of one hour, 118.2 g of aqueous 60% potassium hydroxide solution which has been heated to from 75 to 80°C are added dropwise. The reaction mixture is then maintained at from 95 to 100°C for 4 hours. After cooling to from 70 to 75°C, extraction is carried out with chlorobenzene (first extraction: 1 x 225 g, second and third extraction each 112 g). Yield: 48.5 g of [1,4,5]-oxadiazepine in the extract, corresponding to 86.4% of theory.

Batch 2: Using half of the triple-extracted aqueous phase (containing 1.05 g of the title compound) from batch 1 as the initial charge, 114.0 g of 4,5-diacetyl-[1,4,5]-oxadiazepine (content 89.5%) are introduced at from 90 to 95°C and, in the course of one hour, 118.2 g of aqueous 60% potassium hydroxide solution which has been heated to from 75 to 80°C are added dropwise. The reaction mixture is then maintained at from 95 to 100°C for 4 hours. After cooling to from 70 to 75°C, extraction is carried out. First extraction: combined second and third chlorobenzene extract from batch 1 (containing 9.3 g of the title compound); second and third extraction: each with 112 g of fresh chlorobenzene. Yield: 52.7 g of [1,4,5]-oxadiazepine in the extract, corresponding to 94.1 % of theory.

### Example 4: Preparation of [1,4,5]-oxadiazepine

A mixture of 35.2 g of water, 205 g of chlorobenzene, 100 g of potassium acetate and 96.6 g of 4,5-diacetyl-[1,4,5]-oxadiazepine (96.5% content) is heated to from 90 to 95°C. At that temperature, 107 g of aqueous 60% potassium hydroxide solution which has been heated to from 75 to 80°C are added dropwise in the course of 10 minutes. The reaction mixture is then maintained at from 90 to 100°C for 4 hours. After cooling to from 70 to 75°C, the phases are separated and the aqueous phase is then extracted twice using 100 g of chlorobenzene each time. Yield: 42.8 g of [1,4,5]-oxadiazepine in the extract, corresponding to 83.8% of theory.

### Example 5: Preparation of 4,5-diacetyl-[1,4,5]-oxadiazepine

A mixture consisting of 792 g of dimethyl sulfoxide, 140 g of N,N'-diacetylhydrazine (content 99.5%), 33 g of potassium carbonate, 142 g of potassium hydroxide (content 95%) and 6.6 g of tetramethylammonium chloride is prepared at from 80 to 85°C and evacuated to from 20 to 40 mbar. Under that vacuum and at the same temperature, 258 g of 2,2'-dichlorodiethyl ether are added dropwise in the course of 2 hours and the reaction mixture is then maintained under those conditions for 3 hours. During the dropwise addition and the maintenance period, the water formed under the reaction conditions is removed by distillation. After cooling to from 20 to 25°C, inorganic salt is filtered off, the filtrate is concentrated by evaporation and the residue is crystallised from 1-pentanol. 125.6 g of 4,5-diacetyl-[1,4,5]-oxadiazepine having a content of 93% are obtained, which corresponds to a yield of 52.3%.

### Example 6: Preparation of N,N'-diacetylhydrazine

In the course of 3 hours, at from 40 to 45°C, 191 g of acetic anhydride are metered into 279 g of a solution of 133.4 g of monoacetylhydrazine, 3.8% N,N'-diacetylhydrazine, 18% water, with the remainder being ethanol/ethyl acetate, and then the reaction mixture is maintained at the same temperature for 1 hour. All solvent is then distilled off with a gradual increase in temperature to from 165 to 170°C and a simultaneous reduction in pressure to from 10 to 20 mbar. The residue, 208 g, contains >98% N,N'-diacetylhydrazine, which corresponds to a yield of >98%.

### Example 7: Preparation of [1,4,5]-oxadiazepine

A mixture of 18.6 g of 4,5-diacetyl-[1,4,5]-oxadiazepine (100%), 0.54 g of tetramethylammonium chloride and 100 g of sulfolane is heated to Ti=120-125°C. In the course of 30 min., 4.0 g of potassium hydroxide (95%) are added and the reaction mixture is maintained at that temperature. 0.50 g of water is then added. After a further addition of 8.0 g of potassium hydroxide (95%) over a period of two hours, the reaction mixture is maintained at constant temperature for a further three hours. The reaction mixture is then cooled to room temperature and filtered, and the residue is subsequently washed with sulfolane. The sulfolane filtrate obtained (weight 214.9 g) has a content of 1.74%, which corresponds to a yield of 3.74 g/100% or 38.1 % of theory.

### Example 8: Preparation of [1,4,5]-oxadiazepine

A mixture of 10.7 g of 4,5-dipropionyl-[1,4,5]-oxadiazepine (100%) and 2.0 g of water is prepared at from 95 to 100°C. 12.9 g of potassium hydroxide (50%) are metered in over the course of one hour and the mixture is then stirred for two hours.

In order to complete the reaction, 0.27 g of tetramethylammonium chloride is added, a further 8.0 g of potassium hydroxide (95%) is metered in and then stirring is carried out at from 95 to 110°C for five hours. 7.0 g of chlorobenzene and 10.0 g of water are then added to the reaction mixture at 90°C and the phases are separated at 70°C.

Aqueous phase: 32.5 g having a content of [1,4,5]-oxadiazepine of 2.82%, which corresponds to a yield of 18.0% of theory.

Chlorobenzene phase: 13.0 g having a content of [1,4,5]-oxadiazepine of 10.45%, which corresponds to a yield of 26.6% of theory.

### Example 9: Preparation of [1,4,5]-oxadiazepine

A mixture of 15.53 g of 4,5-dibenzoyl-[1,4,5]-oxadiazepine (100%) and 168.0 g of water is prepared at from 95 to 100°C, 2.0 g of potassium hydroxide (95%) are metered in and then stirring is carried out for one hour. In order to complete the reaction, 0.27 g of tetramethylammonium chloride is added, a further 18.34 g of potassium hydroxide (95%) is metered in over the course of several hours and then stirring is carried out at from 95 to 110°C for a further five hours. The reaction mixture is then cooled to room temperature, filtered and subsequently washed with 200.0 g of water.

Product filtrate: 276.8 g having a content of [1,4,5]-oxadiazepine of 0.62%, which corresponds to a yield of 33.6% of theory.

### Example 10: Preparation of [1,4,5]-oxadiazepine

A mixture of 11.6 g of 6,7,9,10-tetrahydro-8-oxa-5a,10a-diazacyclohepta[b]naphthalene-5,11-dione (100%) and 23.0 g of water is prepared at from 95 to 100°C and 6.78 g of potassium hydroxide (95%) are metered in over the course of several hours. In order to complete the reaction, 0.27 g of tetramethylammonium chloride is added, a further 13.56 g of potassium hydroxide (95%) is metered in over the course of several hours and then stirring is carried out at from 95 to 110°C for a further five hours. In order that the reaction mixture remains stirrable, altogether a further 25 g of water is added.

For working up, 28.0 g of chlorobenzene and 45 g of water are added at 95°C. The emulsion formed is cooled and is analysed without being separated.

Chlorobenzene / water emulsion: 152.8 g having a content of [1,4,5]-oxadiazepine of 1.95%, which corresponds to a yield of 58.4% of theory.

### Example 11: Preparation of [1,4,5]-oxadiazepine

A mixture of 210.9 g of pentanol-moistened 4,5-diacetyl-[1,4,5]-oxadiazepine (186.2 g - 100%) and 42.9 g of water is heated to Ti=100-105°C. Under vacuum, all the water and 1-pentanol is distilled off. At the same temperature, 184.0 g of sodium hydroxide solution (50%) are added in the course of 1 hour. During the sodium hydroxide addition, in parallel 36.8 g of water are added in order to keep the reaction mixture in solution. After a subsequent stirring time of one hour, the reaction mixture is cooled to Ti=90-95°C, 410 g of chlorobenzene are added and the phases are separated at Ti=90°C.

Aqueous phase: 420.0 g having a content of [1,4,5]-oxadiazepine of 5.38%, which corresponds to a yield of 22.1 % of theory.

Chlorobenzene phase: 484.0 g having a content of [1;4,5]-oxadiazepine of 10.91 %, which corresponds to a yield of 51.7% of theory.

### Example 12: Preparation of [1,4,5]-oxadiazepine

A mixture of 210.9 g of pentanol-moistened 4,5-diacetyl-[1,4,5]-oxadiazepine (186.2 g - 100%) and 42.9 g of water is heated to Ti=100-105°C. Under vacuum, all the water and 1-pentanol is distilled off. At the same temperature, 550 g of lithium hydroxide solution (10%) are added in the course of one hour.

After a subsequent stirring time of nine hours, the reaction mixture is cooled to Ti=90-95°C, 410 g of chlorobenzene are added and the phases are separated at Ti=90°C. Aqueous phase: 708.4 g having a content of [1,4,5]-oxadiazepine of 1.18%, which corresponds to a yield of 8.2% of theory.

Chlorobenzene phase: 424.0 g having a content of [1,4,5]-oxadiazepine of 12.34%, which corresponds to a yield of 51.2% of theory.

## Claims

1. A process for the preparation of a [1,4,5]-oxadiazepine derivative, which comprises reacting a 4,6-diacyl-[1,4,5]-oxadiazepine with a base in a polar solvent and at elevated temperature.

2. A process according to claim 1, wherein the base used is an alkali metal hydroxide.

3. A process according to claim 1, wherein the reaction is carried out in the presence of a salt that is soluble in the reaction mixture.

## Patentansprüche

1. Verfahren zur Herstellung von einen [1,4,5]-Oxadiazepin-Derivat, das Umsetzen eines 4,5-Diacyl-[1,4,5]-oxadiazepins mit einer Base in einem polaren Lösungsmittel und bei erhöhter Temperatur umfasst.

2. Verfahren nach Anspruch 1, wobei die verwendete Base ein Alkalimetallhydroxid ist.

3. Verfahren nach Anspruch 1, wobei die Reaktion in Gegenwart von einem Salz, das in dem Reaktionsgemisch löslich ist, ausgeführt wird.

## Revendications

1. Procédé de préparation d'un dérive de la [1,4,5]-oxadiazépine, comprenant la réaction d'une avec une base dans un solvant: polaire et à une température élevée.

2. Procédé selon la revendication 1, dans lequel la base utilisée est un hydroxyde de métal alcalin.

3. Procédé selon la revendication 1, dans lequel la réaction est réalisée en présence d'un sel qui est soluble dans le mélange réactionnel.
